(19) Europäisches Patentamt
European Patent Office
Office européen des brevets

(11) **EP 2 717 890 B1**

(12) **EUROPÄISCHE PATENTSCHRIFT**

(45) Veröffentlichungstag und Bekanntmachung des Hinweises auf die Patenterteilung:
**10.08.2016  Patentblatt 2016/32**

(21) Anmeldenummer: **12733436.5**

(22) Anmeldetag: **08.06.2012**

(51) Int Cl.:
*A61K 35/74* [(2015.01)]    *A61P 31/04* [(2006.01)]

(86) Internationale Anmeldenummer:
**PCT/EP2012/060948**

(87) Internationale Veröffentlichungsnummer:
**WO 2012/168468 (13.12.2012 Gazette 2012/50)**

(54) **SPRÜHGETROCKNETE LACTOBACILLUS STÄMME / ZELLEN UND DEREN VERWENDUNG GEGEN HELICOBACTER PYLORI**

SPRAY-DRIED LACTOBACILLUS STRAINS / CELLS AND THE USE OF SAME AGAINST HELICOBACTER PYLORI

SOUCHES / CELLULES DE LACTOBACILLE SÉCHÉES PAR PULVÉRISATION ET LEUR UTILISATION CONTRE L'HELICOBACTER PYLORI

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.06.2011   EP 11169137**
**19.10.2011   EP 11185851**

(43) Veröffentlichungstag der Anmeldung:
**16.04.2014   Patentblatt 2014/16**

(73) Patentinhaber: **OrganoBalance GmbH**
**13355 Berlin (DE)**

(72) Erfinder:
• **ARYA, Stefanie**
**13347 Berlin (DE)**
• **GOELLING, Detlef**
**25856 Hattstedt (DE)**
• **HOLZ, Caterina**
**10405 Berlin (DE)**
• **LANG, Christine**
**14057 Berlin (DE)**

(74) Vertreter: **Simandi, Claus**
**Simandi Patentanwälte**
**Höhenstrasse 26**
**53773 Hennef (DE)**

(56) Entgegenhaltungen:
EP-A1- 1 112 692          WO-A1-2007/073709
WO-A1-2008/039531          WO-A1-2008/060198

• **TO B C S ET AL: "SPRAY DRYING, FREEZE DRYING, OR FREEZING OF THREE DIFFERENT LACTIC ACID BACTERIA SPECIES", JOURNAL OF FOOD SCIENCE, WILEY-BLACKWELL PUBLISHING, INC, US, Bd. 62, Nr. 3, 1. Januar 1997 (1997-01-01) , Seiten 576-578,585, XP002038914, ISSN: 0022-1147, DOI: 10.1111/J.1365-2621.1997.TB04434.X**
• **SERVIN A L: "Antagonistic activities of lactobacilli and bifidobacteria against microbial pathogens", FEMS MICROBIOLOGY REVIEWS, ELSEVIER, AMSTERDAM, NL, Bd. 28, Nr. 4, 1. Oktober 2004 (2004-10-01), Seiten 405-440, XP004564881, ISSN: 0168-6445**
• **NAOMI UEMURA ET AL: "Helicobacter pylori Infection and the Development of Gastric Cancer", NEW ENGLAND JOURNAL OF MEDICINE, Bd. 345, Nr. 11, 13. September 2001 (2001-09-13), Seiten 784-789, XP55037354, ISSN: 0028-4793, DOI: 10.1056/NEJMoa001999**
• **SOBALA G M ET AL: "Acute Helicobacter pylori infection: clinical features, local and systemic immune response, gastric mucosal histology, and gastric juice ascorbic acid concentrations.", GUT NOV 1991 LNKD- PUBMED:1752479, Bd. 32, Nr. 11, November 1991 (1991-11), Seiten 1415-1418, XP002682923, ISSN: 0017-5749**
• **LORCA GRACIELA L ET AL: "Lactobacillus acidophilus autolysins inhibit helicobacter pylori in vitro", CURRENT MICROBIOLOGY, SPRINGER, NEW YORK, NY, US, Bd. 42, 1. Januar 2001 (2001-01-01), Seiten 39-44, XP002970548, ISSN: 0343-8651, DOI: 10.1007/S002840010175**

- **B.M. Corcoran ET AL: "Comparative survival of probiotic lactobacilli spray-dried in the presence of prebiotic substances", Journal of Applied Microbiology, vol. 96, no. 5, 1 May 2004 (2004-05-01), pages 1024-1039, XP055091314, ISSN: 1364-5072, DOI: 10.1111/j.1365-2672.2004.02219.x**

## Beschreibung

Die Erfindung betrifft sprühgetrocknete Lactobacillus Stämme bzw. Lactobacillus Zellen (Milchsäurebakterien) sowie deren Verwendungen, insbesondere für pharmazeutische und/oder dietätische Zusammensetzungen, einschließlich eines Arzneimittels oder Nahrungsergänzungsmittels, zur Behandlung und Prophylaxe von Helicobacter pylori Infektionen an Mensch und Tier.

Probiotische Mikroorganismen umfassen Zellen, welche vorteilhafte Wirkungen in menschlichen oder tierischen Körpern zeigen. Probiotische Zusammensetzungen enthalten solche Mikroorganismen. Vorteilhafte Wirkungen können insbesondere in der Verbesserung der Mikroflora des Verdauungstraktes bestehen. Insbesondere können in der Mikroflora unerwünschte andere Mikroorganismen durch unmittelbare Wechselwirkungen zwischen den probiotischen Mikroorganismen und den unerwünschten Mikroorganismen, durch mittelbare Wechselwirkungen auf Grund von Hemmungen des Metabolismus des unerwünschten Mikroorganismus durch Expressionsprodukte des probiotischen Mikroorganismus, oder durch Verstärkung des natürlichen Immunsystems gehemmt werden. Allgemein wird angenommen, dass ein Hauptmechanismus die kompetitive Besiedlung des Gastrointestinaltraktes ein wesentliches Wirkelement ist, wodurch unerwünschte Mikroorganismen die Schleimhaut (Mucosa) nicht mehr in störendem Maße besiedeln können bzw. verdrängt werden.

Eine Gruppe probiotischer Mikroorganismen wird beispielsweise durch Laktobazillenstämme gebildet. Hierbei handelt es sich typischerweise um gram-positive, mikroaerophile oder anaerobe Bakterien, welche Zucker fermentieren unter Bildung von Säuren, insbesondere von Milchsäure.

Aus US 5,716,615 ist eine pharmazeutische Zusammensetzung bekannt, welche unter anderem Laktobazillen enthält. Diese ist unter anderem einsetzbar zur Behandlung von Erkrankungen des Gastrointestinaltraktes.

Aus WO 2004/087891 sind Lactobacillus Stämme bekannt, welche zur Herstellung von pharmazeutischen oder dietätischen Zusammensetzungen zur Behandlung von Infektionen des Gastrointestinaltraktes mit Helicobacter pylori geeignet sind.

Wechselwirkungen von Laktobazillen mit Helicobacter pylori sind u. a. beschrieben in Wang et al., Am. J. Clin. Nutr. 80:737-41 (2004), Felley et al., Best Practice & Research Clinical Gastroenterology 17 (5): 785-791 (2003), Cazzato et al., Scandinavian Journal of Nutrition 48(1): 26-31 (2004) und Sgouras et al., Applied and Environmental Microbiology 70 (1): 518-526 (2004).

EP 1 112 692 A1 offenbart sprühgetrocknete Produkte, wie Nahrungsmittel, die Lactobacillus Zellen enthalten können, jedoch keine sprühgetrockneten Lactobacillus Zellen.

WO 2008/060198 A1 offenbart die Verwendung von Lactobacillus Zellen, wie DSM 17581, zur pharmazeutischen Verwendung, wobei die Bakterien auch sprühgetrocknet sein können. Jedoch wird die Behandlung von H. pylori Infektionen nicht offenbart.

Helicobacter pylori ist eine spiralförmige Bakterie, die den Magen kolonisiert, wobei mittels Produktion von Urease der pH Wert im Magen angehoben und in dieser Weise die Bakterien vor der Magensäure geschützt werden. Die Bakterien penetrieren in die Schleimhaut und lagern sich an den Epithelzellen an. Eine solche Infektion aktiviert das körpereigene Immunsystem, wobei die Immunantwort jedoch nicht hinreichend effektiv zur Beseitigung der Infektion ist, mit der Folge einer sich verstärkenden Immunantwort, die zu einer chronischen Entzündung und Erkrankung wie Gastritis bzw. Magengeschwüren und schließlich zum Karzinom führt.

Wenn Zellen untereinander binden und Agglomerate bilden, bezeichnet man diesen Vorgang als Aggregation. Wenn bei dieser Aggregat-Bildung nur eine Zellart beteiligt ist, wird das als Autoaggregation oder Selbstaggregation bezeichnet. Sind bei der Aggregat-Bildung mindestens zwei verschiedene Zellarten beteiligt, bezeichnet man den Vorgang als Co-Aggregation. WO 2007/073709 der Anmelderin beschreibt Co-Aggregate von Laktobazillen mit Helicobacter pylori, welche zur Prophylaxe, Behandlung und/oder einer Eradikationtherapie von Helicobacter pylori Infektionen genutzt werden können, insbesondere wird zumindest eine Reduktion von Helicobacter pylori erreicht. Ferner werden in WO 2007/073709 hierzu geeignete Lactobacillus Stämme beschrieben, die an der Deutschen Sammlung von Mikroorganismen und Zellkulturen GmbH (DSMZ), Mascheroder Weg 1b, D-38124 Braunschweig, Deutschland, hinterlegt wurden und zwar: DSM 17646, DSM 17647, DSM 17648, DSM 17649, DSM 17650, DSM 17651, DSM 17652 und DSM 17653.

Die geeigneten Lactobacillus Zellen bilden beim Kontakt mit Helicobacter pylori Zellen Co-Aggregate. Durch die Bildung von Co-Aggregaten wird Helicobacter pylori daran gehindert, in die Magenschleimhaut einzudringen. Die Helicobacter pylori Zellen, insbesondere deren Zelloberfläche, werden durch die Lactobacillus Zellen maskiert, so dass die Helicobacter pylori Zellen nicht mehr in der Lage sind, an die Magenepithelzellen zu binden. Durch die verhinderte Bindung von Helicobacter pylori an die Magenepithelzellen bleiben Entzündungsreaktionen aus. Die maskierten und damit deaktivierten Helicobacter pylori Zellen werden in Form von Co-Aggregaten durch den Magen-Darm-Trakt geschleust und ausgeschieden. Es ist also möglich, durch die Gabe von geeigneten Lactobacillus Zellen die Helicobacter pylori Zellen im Magen zu reduzieren bzw. zu eradizieren. Die Anwendung der Lactobacillus Zellen kann prophylaktisch oder kurativ erfolgen. Allerdings gilt es, diesen Therapieansatz zu verbessern.

Daher liegt der Erfindung die Aufgabe zugrunde, eine verbesserte Zusammensetzung zur Prophylaxe und Behandlung von Helicobacter pylori Infektionen bereitzustellen. Insbesondere eine solche Zusammensetzung oder Mittel, das eine verbesserte Co-Aggregatbildung erlaubt, wobei mittels ausgebildeten Co-Aggregaten die Besiedlung der Magenschleimhaut mit Helicobacter pylori deutlich besser inhibiert/verringert wird.

Der Einsatz und die Herstellung von sprühgetrockneten Laktobazillen im Stand der Technik ist bekannt, z. B. Gardiner et al., Comparative Survival Rates of Human-Derived Probiotic Lactobacillus paracasei and L. salivarius Strains during Heat Treatment and Spray Drying, Applied and Enviromental Microbiology 66 (6): 2605-2612 (2000) und Teixeira et al., Survival of Lactobacillus delbrueckii ssp. Bulgaricus Following Spray-Drying, J. Dairy Sci 78:1025-1031 (1995) und Tos t al., Spray Drying, Freeze Drying, or Freezing of three different lactic acid bacteria species, Journal of Food Sci., Wiley-Blackwell Publ., Inc. (US).

Nicht beschrieben ist jedoch die Verwendung von sprühgetrockneten Laktobazillen zur Bildung von Co-Aggregaten mit Helicobacter pylori und deren besonderen Eignung in Form einer pharmazeutischen oder diätischen Zusammensetzung.

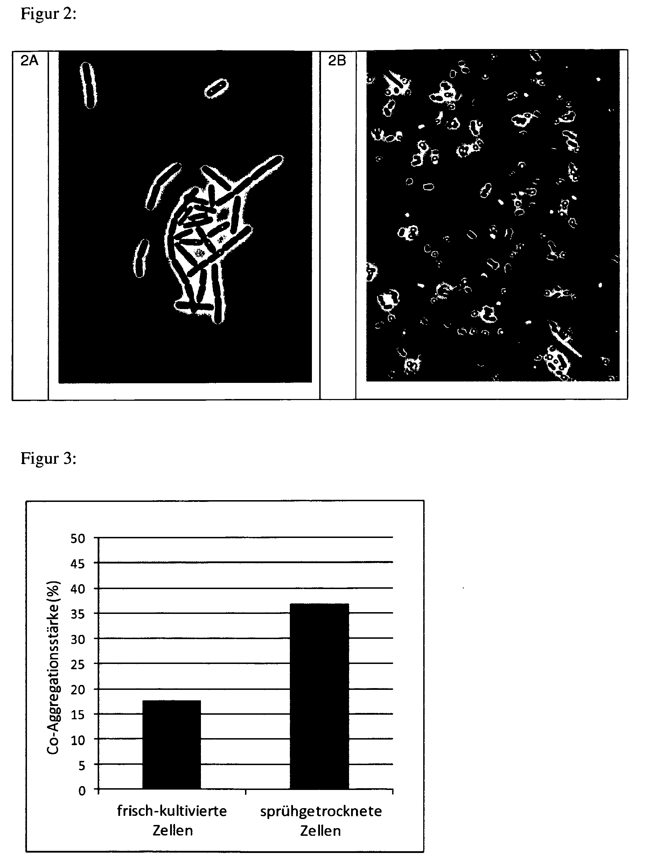

Sprühgetrocknete Lactobacillus Zellen können besonders vorteilhaft große Co-Aggregate mit Helicobacter pylori bilden und auf diese Weise eine effiziente Reduzierung der Keimbelastung durch Helicobacter pylori bewirken. Besonders vorteilhaft führt die Sprühtrocknung der Lactobacillus Zellen zu einer Verkleinerung der Lactobacillus Zellen, wobei zudem eine Vereinzelung / Separation der Zellen erfolgt. Im Gegensatz zu anderen Trocknungsverfahren oder ohne Trocknung durchgeführten Verfahren werden keine Ketten von zwei bis zehn Lactobacillus Zellen erhalten (Figur 2A), sondern - für die Sprühtrocknung charakteristisch - einzelne Zellen (Mono) oder Zweierzellen (Dimer) (Figur 2B). Diese sprühgetrockneten "Keimzellen" eignen sich hervorragend zur in-situ Bildung der Co-Aggregate nach Applikation im Magenmedium und es werden vorteilhafte hochdichte, kompakte und effiziente Co-Aggregate aus Lactobacillus Zellen und Helicobacter pylori (Zellen) erhalten, die zudem eine vorteilhafte geringe sterische Hinderung in der Bildungsphase der Co-Aggregate aufweisen. Solche erfindungsgemäßen sprühgetrockneten Lactobacillus Zellen weisen eine höhere Bindungsaffinität an Helicobacter pylori auf, als beispielsweise jene die gemäß der technischen Lehre aus WO 2007/073709 hergestellt werden können. Durch die höhere Bindungsaffinität können durch weniger Laktobazillen Zellen mehr Helicobacter pylori Zellen maskiert und gebunden werden. Die höhere Bindungsaffinität führt weiterhin zu einer höheren Stabilität der gebildeten Co-Aggregate.

Weiterhin ist vorteilhaft, dass sprühgetrocknete Lactobacillus Zellen ebenfalls einen erhöhten Anteil an nichtlebenden und / oder Fragmenten von Lactobacillus Zellen aufweisen, welche ebenfalls die spontane Co-Aggregat Bildung unterstützen. Der Anteil an nicht-lebenden und / oder Fragmenten von Lactobacillus Zellen kann mehr als 80 %, mehr als 90 % gar 100 % betragen.

Die erfindungsgemäßen Co-Aggregate durchlaufen den Gastrointestinaltrakt und verlassen den Körper auf natürlichem Wege. Selbst bei bereits erfolgter Infektion ist dieser Wirkmechanismus erfindungsgemäßer sprühgetrockneter Lactobacillus Stämme hilfreich, da eine weitere Infektion mit zusätzlichen Helicobacter pylori Bakterien verhindert wird und so die bestehende Infektion durch Inaktivierung / Ausscheidung der vorhandenen Helicobacter pylori Bakterien leichter bekämpft werden kann. Hinzu kommt, dass erfindungsgemäße Lactobacillus Stämme vermutlich auch zur Hemmung der Urease Aktivität von Helicobacter pylori in der Lage sind, so dass die Helicobacter pylori Bakterien in den Co-Aggregaten ihren Schutz gegen den Angriff von Magensäure verlieren. Insofern wird auch ein synergistischer Effekt erzielt.

Sprühgetrocknete Lactobacillus Stämme oder Lactobacillus Zellen weisen den besonderen Vorteil auf, dass die Bindungsaffinität zu Helicobacter pylori erhöht ist (supra). Weil die Oberfläche von Helicobacter pylori durch die Lactobacillus Zellen maskiert ist, ist eine Penetration von Helicobacter pylori in die Schleimhaut verhindert und folglich kann eine Helicobacter pylori Infektion samt chronischen Entzündungsprozessen nicht eintreten und Folgeerkrankungen wie Gastritis, Magengeschwüre oder Ulcus bis hin zum Magenkrebs werden sicher verhindert.

Darüber hinaus weisen die erfindungsgemäßen Co-Aggregate aus sprühgetrockneten Lactobacillus Zellen und Helicobacter pylori Zellen eine erhöhte Stabilität auf, so dass erstens eine erhöhte Zahl von Helicobacter pylori in diese Co-Aggregate eingebunden werden kann und zweitens keine Wiederfreisetzung von vorher gebundenen Helicobacter pylori Zellen durch z. B. Magenbewegungen stattfindet. Dieser Vorteil erlaubt gegenüber den im Stand der Technik bekannten Co-Aggregaten eine vorteilhafte niedrigere Dosierung. Ferner erlauben die erfindungsgemäßen sprühgetrockneten Lactobacillus Zellen eine vorteilhafte erhöhte Löslichkeit in Wasser, so dass eine verbesserte Verteilung der sprühgetrockneten Lactobacillus Zellen und der erhaltenen Co-Aggregate im Magenraum erreicht werden kann.

Ferner konnten die Erfinder feststellen, dass durch die Sprühtrocknung der Lactobacillus Zellen eine unkontrollierte Aggregatbildung der Laktobazillen selbst (Autoaggregation) verhindert oder gegenüber frischen nicht-sprühgetrockneten Lactobazillen zumindest stark reduziert ist. Durch Autoaggregation der Lactobacillus Zellen untereinander werden die Bindungsstellen für die Maskierung von Helicobacter pylori besetzt. Die Reduzierung bzw. Verhinderung der Autoaggregation führt also ebenfalls zu einer vorteilhaften niedrigeren Dosierung der eingesetzten Laktobazillen gegenüber dem Stand der Technik.

Das erfindungsgemäße Sprühverfahren bewirkt eine morphologische Änderung der Laktobazillen, so dass die Bindungsaffinität für Helicobacter pylori erhöht ist und damit eine verbesserte Co-Aggregatbildung erfolgen kann.

Das Sprühverfahren bewirkt eine morphologische Änderung der Laktobazillen, so dass die Bindungsaffinität für Helicobacter pylori erhöht ist und damit eine verbesserte Co-Aggregatbildung erfolgen kann.

Daher betrifft die Erfindung eine pharmazeutische oder diätische Zusammensetzung enthaltend sprühgetrocknete Lactobacillus Zellen zur Verwendung in der Prophylaxe und Behandlung von Helicobacter pylori Infektionen an Mensch und Tier, insbesondere Säugetier.

In einer weiteren Ausführungsform der Erfindung liegen die sprühgetrockneten Lactobacillus Zellen in der Zusammensetzung vorzugsweise und im Wesentlichen in einer mono und /oder dimeren Form vor (supra, Fig. 2B).

Daher kann eine solche erfindungsgemäße Zusammensetzung hergestellt werden, wobei a.) Lactobacillus Zellen sprühgetrocknet werden und b.) erhaltene sprühgetrocknete Lactobacillus Zellen in einen physiologisch verträglichen Träger eingebracht werden (Beispielhafte Ausführungsformen von physiologisch verträglichen Trägern sind unten beschrieben).

Weiterhin betrifft die Erfindung eine solche Zusammensetzung, wobei nach Applikation an Mensch oder Tier Co-Aggregate mit Helicobacter pylori im Magenmedium in-situ gebildet werden, die vorzugsweise größer und nicht kleiner als 50 $\mu$m, insbesondere größer und nicht kleiner als 100 $\mu$m, 150 $\mu$m, insbesondere größer als 500 $\mu$m, besonders bevorzugt größer als 1.000 $\mu$m oder 1.100 $\mu$m sind.

Der Begriff "Lactobacillus Zellen" iSd. Erfindung (iwS. Milchsäurebakterien, auch Lactobazillen) umfasst solche Mikroorganismen, die Kohlenhydrate, insbesondere Glukose und Lactose, zur Milchsäurevergärung benötigen und zumeist den Embden-Meyerhof-Biosyntheseweg nutzen. Die Lactobacillus Zellen werden taxonomisch in der Familie Lactobacteriaceae zusammengefasst. Sie sind gram-positiv, nicht sporenbildend und im Allgemeinen unbeweglich. Die Lactobacillus Zellen leben anaerob, sind jedoch aerotolerant, obwohl sie keine Hämine (Cytochrom, Katalase) enthalten (Schleifer et al., System. Appl. Microb.: 18, 461-467 (1995) oder Ludwiq et al., System. Appl. Microb. 15: 487-501 (1992). Die Lactobacillus Zellen bzw. die Spezies können auf der Grundlage des *acidophilus, Lactobacillus bulgaricus, Lactobacillus amylovorus*, *Lactobacillus delbrueckii, Lactobacillus casei, Lactobacillus crispatus, Lactobacillus gasseri, Lactobacillus johnsonii, Lactobacillus paracasei, Lactobacillus pentosus, Lactobacillus rhamnosus, Lactobacillus curvatus* und *Lactobacillus plantarum* (alle homofermentativ), weiterhin *Lactobacillus brevis, Lactobacillus buchneri, Lactobacillus fructivorans, Lactobacillus hilgardii, Lactobacillus fermentum, Lactobacillus reuteri, Lactobacillus viridescens* als auch *Bifidobacterium bifidum* (alle heterofermentativ). Beispielhafte geeignete Lactobacillus Zellen der Anmelderin sind hinterlegt: DSM 17646, DSM 17647, DSM 17648, DSM 17649, DSM 17650, DSM 17651, DSM 17652 und DSM 17653 (supra). *Lactobacillus fermentum, Lactobacillus brevis, Lactobacillus reuteri, Lactobacillus buchneri und Lactobacillus pentosus* sind erfindungsgemäß bevorzugt.

Erfindungsgemäß umfasst sind ebenfalls solche "Lactobacillus Zellen" einschließlich "Derivate" oder "Analoga" oder "Mutanten" hiervon, die inaktiviert, lebend oder nicht-lebend sind oder Teile und Fragmente, z.B. enzymatische oder mechanische Spaltprodukte (z.B. French Press etc.), von Lactobacillus Zellen darstellen soweit diese die Eignung zur Co-Aggregation aufweisen.

Die Begriffe und "Derivate", "Analoga""Mutanten" oder "inaktiviert" schließen vorliegend auch Zell- bzw. Fermentationsüberstände, Lysate, Fraktionen oder Extrakte der "Lactobacillus Zellen" mit ein, wobei diese Zell- bzw. Fermentationsüberstände, Lysate, Fraktionen oder Extrakte bevorzugt die Eigenschaften der erfindungsgemäßen Lactobacillus Zellen /Stämme / Mikroorganismen besitzen. Hierbei bedeutet "Lysat" - wie auch der Begriff "Extrakt" - insbesondere eine Lösung oder Suspension in einem wässrigem Medium der erfindungsgemäßen Zellen des Mikroorganismus und umfasst bspw. Makromoleküle, wie DNA, RNA, Proteine, Peptide, Lipide, Kohlenhydrate u. ä., sowie insbesondere auch Zelltrümmer. Vorzugsweise umfasst das Lysat auch Zellwand oder Zellwandbestandteile. Verfahren zur Herstellung von Lysaten sind dem Fachmann hinreichend bekannt und umfassen z.B. den Einsatz einer "French Press" oder enzymatische Lyse, Kugelmühle mit Glasperlen oder Eisenkugeln. Das Aufbrechen von Zellen kann enzymatisch, physikalisch oder auch chemisch erfolgen. Beispiele für enzymatische Zelllyse können einzelne Enzyme wie auch Enzymcocktails sein, wie Proteasen, Proteinase K, Lipasen, Glykosidasen; Chemische Lysen können bewirkt werden durch Ionophoren, Detergenzien, wie SDS, Säuren oder Basen; Physikalischen Methoden können durch hohe Drücke, wie French Press, Osmolaritäten, Temperaturen oder Wechsel zwischen Hitze und Kälte bewirkt werden. Ferner können natürlich chemischen, physikalischen und enzymatische Methoden kombiniert werden.

"Derivate" oder "Analoga" oder "Mutanten" oder "inaktiviert" der erfindungsgemäßen Lactobacillus Zellen / Stämme / Mikroorganismen weisen vorzugsweise die gleichen Eigenschaften, wie die benannten Stämme auf. Dabei ist eine metabolische Aktivität bei der "inaktivierten (Form)" oder "Derivate" oder "Analoga" vorzugsweise nicht mehr gegeben. "Analoga" der erfindungsgemäßen Lactobacillus Zellen / Stämme / Mikroorganismen stellen eine Form des Lysates oder Fragmente dar. Ein "Fragment" der erfindungsgemäßen Lactobacillus Zellen / Stämme / Mikroorganismen stellt einen Teil der Zellen dar, wie z.B. Zellmembran, Makromoleküle, wie DNA, RNA, Proteine, Peptide, Lipide, Kohlenhydrate u.ä., sowie auch Zelltrümmer. Der Fachmann kann die Begriffe "Analoga", Fragmente", "Derivat" oder "Mutante" mit sinngemäßem Inhalt belegen und die Begriffe im Sinne der vorliegenden Erfindung auslegen. Um Mutanten, Derivate, Fragmente oder Analoga von den bevorzugten Lactobacillus Zellen / Stämme / Mikroorganismen bereitzustellen, kann der Fachmann auf Standardliteratur zurückgreifen, die ihm vorliegt und Techniken offenbart, die zur Herstellung von Mutanten, Derivaten, Fragmenten oder Analoga genutzt werden können. Mutanten bzw. genetisch veränderte Varianten oder Derivate werden genetisch verändert, beispielsweise durch rekombinante DNA-Technologien (Klonen, Sequenzieren, Transformieren rekombinanter Nukleinsäuren), wie auch physikalische Mutagenese, beispielsweise durch ultraviolette Strahlung, aber auch durch chemische Agenzien, wie mit Ethyl-Methansulfonat (EMS). Dabei können Veränderungen in den positiven Eigenschaften selektiert werden- entweder gezielt oder durch Evaluierung einer Vielzahl von entstandenen Mutanten. Genetisch veränderte Mutanten beinhalten Zellen der erfindungsgemäßen Mikroorganismen und beherbergen rekombinate Nukleinsäuren in ihrem bakteriellen Chromosom und/oder Plasmiden. Modifikationen durch Punktmutationen können zudem Auswirkungen auf die Expression/Transkription/Translation bewirken, wie auch spontane Mutationen ohne direkte genetische Manipulation (Siehe z.B. J. Sambrook, E.F. Fritsch, T. Maniatis, Cold Molecular cloning: a laboratory manual / Spring Harbor Laboratory Press, 3. Auflage (2001)).

All diese Mikroorganismen werden nach- und vorstehend "Lactobacillus Zellen" genannt.

Chromosom und/oder Plasmiden. Modifikationen durch Punktmutationen können zudem Auswirkungen auf die Expression/Transkription/Translation bewirken, wie auch spontane Mutationen ohne direkte genetische Manipulation (Siehe z.B. J. Sambrook, E.F. Fritsch, T. Maniatis, Cold Molecular cloning: a laboratory manual / Spring Harbor Laboratory Press, 3. Auflage (2001)).

All diese Mikroorganismen werden nach- und vorstehend "Lactobacillus Zellen" genannt.

Die wesentlichen Kulturbedingungen des menschlichen Magentraktes umfassen einen pH-Wert im Bereich von 1,8 bis 4,5 und die Gegenwart von Pepsin sowie NaCl. Ein Referenzmedium, welches für solche Kulturbedingungen charakteristisch ist, besteht aus den folgenden Komponenten: Wasser, 5 g/l NaCl sowie 3 g/l Pepsin wobei der pH Wert auf 2,0 bzw. 4,0 mit Salzsäure eingestellt ist, um einen leeren bzw. gefüllten Magen zu simulieren.

Der Begriff der "Co-Aggregation" iSd. Erfindung bezeichnet die Bildung von Zellaggregaten einer Größe von zumindest 50 μm oder 100 μm und mehr, enthaltend erfindungsgemäße sprühgetrocknete Lactobacillus Zellen und Helicobacter pylori Zellen, in Suspensionen, beispielsweise gemäß der folgenden Beispiele, insbesondere in einem Referenzmedium, wie vorstehend beschrieben.

Der Begriff "sprühgetrocknete Lactobacillus Zellen" iSd. Erfindung bedeutet, dass die Lactobacillus Zellen mittels einem Sprühtrocknungs- oder Zerstäubungsverfahren (Synonym) getrocknet werden, wobei z.B. eine Suspension von Lactobacillus Zellen in feine nebelartige Tröpfchen zerteilt wird und ein Pulver erhalten werden kann.

Bei einer Sprühtrocknung wird eine Lösung oder Suspension enthaltend Lactobacillus Zellen in ein heißes Trocknungsmedium versprüht und dadurch getrocknet. Das zu versprühende Gemisch kann in Form von Lösung, Emulsion, Suspension oder Dispersion vorliegen. Es wird mit Hilfe einer Düse oder einem Sprührad in Millionen von einzelnen Tröpfchen zerstäubt, wobei die Oberfläche stark vergrößert wird. Das Lösungsmittel, wie Wasser, wird sofort durch die heiße Luft verdampft und abgeführt. Ferner werden die Lactobacillus Zellen alleine sprühgetrocknet. der Firma Büchi Labortechnik AG (Deutschland) oder SD-6.3-R der Firma GEA Niro (Dänemark). Ferner ist bekannt, dass beliebige Hilfs- und Zusatzstoffe verwendet werden können.

Die Erfindung betrifft des Weiteren eine pharmazeutische und/oder diätische Zusammensetzung enthaltend eine physiologisch wirksame Dosis an erfindungsgemäßen sprühgetrockneten Lactobacillus Zellen sowie einen physiologisch verträglichen Träger. Bei pharmazeutischen Zusammensetzungen handelt es sich um Zusammensetzungen, die einzig therapeutischen oder prophylaktischen Zwecken dienen und wobei neben den Lactobacillus Zellen lediglich in der Galenik übliche Hilfs- und/oder Trägerstoffe zugegen sind. Bei diätetischen Zusammensetzungen im Sinne der vorliegenden Erfindung handelt es sich um Zusammensetzungen, welche neben den erfindungsgemäßen sprühgetrockneten Lactobacillus Zellen ein Nahrungs- oder Lebensmittel (siehe z.B. nicht abschließend EU-Richtlinie 2002/46/EG vom 10. Juni 2002) und/oder ein Futtermittel für Haus- und/oder Nutztiere und / oder Nahrungsergänzungsmittel umfassen, ggfs. enthaltend Hilfs- und Zusatzstoffe.

Die Erfindung betrifft auch die Verwendung oder Anwendung von erfindungsgemäßen sprühgetrockneten Lactobacillus Zellen zur Herstellung einer pharmazeutischen oder diätetischen Zusammensetzung, oder ein Arzneimittel oder ein Nahrungsergänzungsmittel enthaltend sprühgetrocknete Lactobacillus Zellen oder eine pharmazeutische oder diätetische Zusammensetzung, insbesondere zur Prophylaxe und/oder Behandlung von durch Infektion mit Helicobacter pylori bedingten Erkrankungen, beispielsweise Gastrointestinalerkrankungen. Hierzu zählen insbesondere Gastritis, Magengeschwüre, Ulcus und Magenkrebs. Weiterhin sind solche Erkrankungen und Symptome umfasst, wie Bauchbeschwerden, insbesondere Oberbauchbeschwerden, Magendruck, Magenkrämpfe, Magenschmerzen, Schmerzen oder Druck im Oberbauch, Brennen im Oberbauch, chronischrezidivierende Abdominalbeschwerden, ständiges Völlegefühl, Appetitlosigkeit, Nüchternschmerz, Blähungen, Sodbrennen, Durchfall, Stuhlunregelmäßigkeiten, Unwohlsein, Brechreiz, Übelkeit und Erbrechen, Speiseunverträglichkeiten, Malabsorption, Reizmagen (funktionelle Dyspepsie), Magenschleimhautentzündung, Schleimhautschädigung, gastroduodenale Ulkuskrankheit.

Weiterhin betrifft die Erfindung die Verwendung oder Anwendung der erfindungsgemäßen sprühgetrockneten Lactobacillus Zellen, oder ein Arzneimittel oder ein Nahrungsergänzungsmittel enthaltend sprühgetrocknete Lactobacillus Zellen oder eine pharmazeutische oder diätetische Zusammensetzung, zur Eradikation bzw. Eradikationstherapie von Helicobacter pylori, ggfs. in Kombination mit weiteren geeigneten Wirkstoffen, wie Antibiotika.

Eine erfindungsgemäße pharmazeutische oder diätische Zusammensetzung, insbesondere ein Nahrungsergänzungsmittel oder Arzneimittel (Medikament), kann dadurch gekennzeichnet sein, dass sie $10^2$ bis $10^{15}$, vorzugsweise $10^4$ oder $10^8$ bis $10^{12}$, insbesondere $10^8$ bis $10^{10}$, sprühgetrocknete Lactobacillus Zellen enthält. Bezugsgröße ist dabei eine Gabeeinheit, beispielsweise eine Tablette. Vorzugsweise ist die Zusammensetzung zur oralen Gabe hergerichtet.

Die galenische Herrichtung einer erfindungsgemäßen pharmazeutischen oder diätischen Zusammensetzung, insbesondere ein Nahrungsergänzungsmittel oder Arzneimittel (Medikament), kann in fachüblicher Weise erfolgen. Geeignete feste oder flüssige galenische Zubereitungsformen sind beispielsweise Granulate, Pulver, Dragees, Tabletten, (Mikro-) Kapseln, Hartkapseln, Suppositorien, Sirupe, Säfte, Suspensionen, oder Emulsionen, bei deren Herstellung übliche Hilfsmittel wie Trägerstoffe, Spreng-, Binde-, Überzugs-, Quellungs-, Gleit™ oder Schmiermittel, Geschmacksstoffe, Süßungsmittel und Lösungsvermittler, Verwendung finden. Als Hilfsstoffe sei Magnesium-Stearat, Natriumchlorid, Magnesiumcarbonat, Titandioxid, Lactose, Mannit und andere Zucker, Talcum, Milcheiweiß, Gelatine, Stärke, Zellulose und ihre Derivate, tierische und pflanzliche Öle wie Lebertran, Sonnenblumen-, Erdnuss- oder Sesamöl, Polyethylenglycole und Lösungsmittel, wie etwa steriles Wasser und ein- oder mehrwertige Alkohole, beispielsweise Glycerin, genannt. Eine erfindungsgemäße pharmazeutische Zusammensetzung ist dadurch herstellbar, dass Zellen mindestens eines erfindungsgemäß verwendeten Lactobacillus Stammes in definierter Dosis mit einem pharmazeutisch geeigneten und physiologisch verträglichen Träger und ggf. weiteren geeigneten Wirk-, Zusatz- oder Hilfsstoffen mit definierter Dosis gemischt und zu der gewünschten Darreichungsform hergerichtet ist. Als Träger kommen insbesondere Stoffe in Frage, die ausgewählt sind aus der Gruppe bestehend aus Maltodextrin, mikrokristalline Zellulose, Stärke, insbesondere Maisstärke, Levulose, Lösungsmittel, wie etwa steriles Wasser und ein- oder mehrwertige Alkohole, beispielsweise Glycerin, genannt. Eine erfindungsgemäße pharmazeutische Zusammensetzung ist dadurch herstellbar, dass Zellen mindestens eines erfindungsgemäß verwendeten Lactobacillus Stammes in definierter Dosis mit einem pharmazeutisch geeigneten und physiologisch verträglichen Träger und ggf. weiteren geeigneten Wirk-, Zusatz- oder Hilfsstoffen mit definierter Dosis gemischt und zu der gewünschten Darreichungsform hergerichtet ist. Als Träger kommen insbesondere Stoffe in Frage, die ausgewählt sind aus der Gruppe bestehend aus Maltodextrin, mikrokristalline Zellulose, Stärke, insbesondere Maisstärke, Levulose, Lactose, Dextrose, und Mischungen solcher Substanzen. Die Zusammensetzung kann 0,1 bis 95 Gew.-% Träger und 5 bis 99.9 Gew.-% sprühgetrocknete Lactobacillus Zellen, bezogen auf die Gesamtmenge an Zellen und Träger, enthalten bzw. hieraus bestehen.

Im Falle der dietätischen Zusammensetzung und / oder Nahrungsergänzungsmittel kann vorgesehen sein, dass die Zusammensetzung $10^2$ bis $10^{15}$, vorzugsweise $10^4$ bis $10^{12}$, insbesondere $10^8$ bis $10^{10}$, Lactobacillus Zellen enthält. Bezugsgröße ist eine Gabeeinheit, beispielsweise eine Verpackungseinheit eines Lebensmittels zum Verkauf an einen Endverbraucher. Der physiologisch verträgliche Träger wird in der Regel ein Lebensmittel sein, welches insbesondere ausgewählt ist aus der Gruppe bestehend aus Milchprodukten, fermentierten Milchprodukten, Milch, Joghurt, Käse, Cerealien, Müsliriegel, Backwaren- und Getränken und Kindernahrungszubereitungen. Geeignete erfindungsgemäße Nahrungs- oder Lebensmittel, einschließlich Wasser, sind solche wie z.B. in der Verordnung (EG) Nr. 178/2002 vom 28. Januar 2002 nicht abschließend definiert.

Die Erfindung betrifft weiterhin ein Verfahren zur Herstellung einer erfindungsgemäßen pharmazeutischen und/oder diätetischen Zusammensetzung, insbesondere ein Nahrungsergänzungsmittel oder Arzneimittel (Medikament), wobei die sprühgetrockneten Lactobacillus Zellen mit dem physiologisch verträglichen Träger gemischt und vorzugsweise zur oralen Gabe hergerichtet werden.

Ferner ist offenbart ein Verfahren zur Herstellung einer erfindungsgemäßen Zusammensetzung, wobei die Lactobacillus Zellen (i) optional angeimft und angereichert (ii) fermentiert und (iii) sprühgetrocknet werden und anschließend mit einem physiologisch verträglichen Träger gemischt und vorzugsweise zur oralen Gabe hergerichtet werden. unbeschränkt. Insbesondere letzteres eignet sich für eine dauerhafte Prophylaxe sowie eine Vorbeuge gegen Rückfallerkrankungen.

Im Folgenden wird die Erfindung anhand von lediglich Ausführungsformen darstellenden Beispielen näher erläutert, ohne die Erfindung auf diese Beispiele beschränken zu wollen.

Beispiele und Figuren:

Beispiel 1: Lagerung verwendeter Stämme

Die Lagerung der Lactobacillus Stämme erfolgte im gefrorenen Zustand. 1 ml einer bis zur stationären Phase ($OD_{600}$/ml 4-8) in MRS Medium (55g/l, pH 6,5; Difco, USA) kultivierten Kultur wurde mit 500 µl einer 50 % igen (v/v) sterilen Glycerinlösung gemischt und die Mischung bei - 80 °C eingefroren.

Die Lagerung von Helicobacter pylori erfolgte in gefrorenem Zustand. 1 ml einer bis zur stationären Phase in Brucella broth (28g/l, pH 7,0; BD, USA), supplementiert mit 10 % (v/v) fetalem Kälberserum (Biochrom), kultivierten Kultur wurde mit 500 µl einer 50 % igen (v/v) sterilen Glycerinlösung gemischt und die Mischung bei - 80 °C eingefroren.

Beispiel 2: Prozess zur Herstellung von sprühgetrockneten Lactobacillus Zellen

Ein sprühgetrockneter Lactobacillus, der in der Lage ist, Helicobacter pylori zu coaggregieren, unter Kulturbedingungen des menschlichen Verdauungstraktes, insbesondere des Magens, wurde wie im folgenden beschrieben hergestellt:

Die Vorkultur 1 fand in einem 15 ml Reaktionsgefäß in 10 ml MRS-Medium statt. Das Medium wurde mit frisch kultivierten, tiefgefrorenen, gefriergetrockneten oder sprühgetrockneten Zellen des Lactobacillus Stamm angeimpft. Diese Vorkultur 1 wurde für 24 h bei 37 °C unter anaeroben Bedingungen inkubiert. Die komplette Vorkultur 1 wurde zum Animpfen der Vorkultur 2 verwendet. Vorkultur 2 bestand aus 240 ml MRS-Medium mit 10 ml der Vorkultur 1. Vorkultur 2 wurde für 19 h bei 37 °C unter anaeroben Bedingungen inkubiert. Am Ende jeder Vorkultur wurden optische Dichte, pH-Wert und cfu bestimmt.

Für die Fermentation von Lactobacillus wurden 5 Liter MRS verwendet. Der Fermenter mit allen erforderlichen Komponenten wurde autoklaviert. Sollwerte für Temperatur und Rührerdrehzahl waren 37 ° C und 150 U / min. Die Animpfkonzentration betrug 5%. Am Ende der Fermentation wurden Zellzahl, Kolonie bildende Einheiten, pH, optische Dichte bei 600 nm und die Konzentration von Glukose und Laktat bestimmt. Im Anschluss wurde die Fermentationsbrühe 10fach bis 20fach aufkonzentriert. Im Anschluss wurde das Konzentrat eingefroren.

Vor der Trocknung wurde das 20-fach-Konzentrat aufgetaut und bei 4300 x g für 15 Minuten zentrifugiert. Die Zellen wurden einmal mit 0,9 % NaCl-Lösung gewaschen und bei 4300 x g für 15 Minuten erneut zentrifugiert. Dann wurden die Zellen in 500 ml 10 % NaCl-Lösung resuspendiert.

Die Sprühtrocknung wurde in einem Büchi Mini Spray Dryer B-191 durchgeführt. Die Eingangstemperatur betrug 140 °C. Die Ausgangstemperatur betrug 86 °C. Der Heißluftstrom betrug 550 L/h. Die Aspirator-Leistung betrug 75 %, die Pumprate 5 %. Die Zellsuspension wurde mit vorstehenden Parametern getrocknet und im Anschluss wurde das sprühgetrocknete Lactobacillus Pulver entnommen.

Daraufhin folgte eine Bestimmung der Gesamtzellzahl sowie Lebendzellzahl des Lactobacillus Stamms pro Gramm sprühgetrocknetem Pulver. Die mikroskopische Untersuchung des sprühgetrockneten Pulvers (Figur 2B) zeigte, dass die Lactobacillus Zellen kleiner sind als nicht-sprühgetrocknete Lactobacillus Zellen (Figur 2A) und dass die sprühgetrockneten Lactobacillus Zellen in Mono- und Dimeren vorliegen, während die nicht-sprühgetrockneten Lactobacillus Zellen längere Ketten aufweisen. Bis zur weiteren Verwendung wurden die sprühgetrockneten Lactobacillus Zellen bei 4 °C gelagert.

Beispiel 3: Vergleich der Co-Aggregation von Helicobacter pylori durch nicht sprühgetrocknete und sprühgetrocknete Lactobacillus Stämme bzw. Lactobacillus Zellen unter Magenbedingungen

Die Kultivierung der Lactobacillus Zellen erfolgte in geschlossenen 15 ml Röhrchen in MRS Medium bei 37 °C für 24 h. Für die Untersuchung von sprühgetrockneten Laktobazillen wurden das sprühgetrocknete Pulver in PBS mit einer Konzentration von 10 mg / ml resuspendiert. Helicobacter pylori wurde für ca. 2 Tage in Erlenmeyerkolben unter mikroaerophilen Bedingungen in Brucella Bouillon (28 g / l, pH 7,0; BD, USA) mit 10% fötalem Kälberserum (Biochrom) bei 37 °C kultiviert. Nach der Kultivierung wurde die Zellmorphologie von Helicobacter pylori mikroskopisch untersucht. Es wurden Assays mit Zellen sigmoidaler Morphologie oder mit Zellen coccoider Morphologie durchgeführt. Auch Kulturen mit gemischter Morphologie wurden untersucht.

Die jeweiligen Zellen wurden durch Zentrifugation bei 3200 g für 10 min. geerntet und der Überstand wurde verworfen. Die Lactobacillus Zellen wurden einmal in 5 ml Puffer gewaschen und in 5 ml PBS Puffer resuspendiert (PBS-Puffer enthaltend 1,5 g/l $Na_2HPO_4*2H_2O$, 0,2 g/l $KH_2PO_4$ und 8,8 g/l NaCl). Die Helicobacter pylori Zellen wurden einmal in 5 ml PBS Puffer gewaschen und in 5 ml künstlichem Magensaft resuspendiert (enthaltend 5 g/l NaCl und 3 g/l Pepsin (Sigma)). Der $OD_{600}$ Wert wurde für die jeweiligen Zellen gemessen und auf einen Wert von 2 für Helicobacter pylori bzw. 4 für Lactobacillus durch Zugabe von künstlichem Magensaft bzw. PBS Puffer eingestellt.

2,5 ml jeder so erhaltenen Zellsuspension (Helicobacter pylori/Lactobacillus) wurden gemischt und die Mischung wurde für 10 s bis 10 min. geschüttelt. Das Ergebnis war sowohl optisch sichtbar durch deutliche Flockulation in den Proben, die Helicobacter pylori und Lactobacillus enthielten, und wurde auch mikroskopisch untersucht.

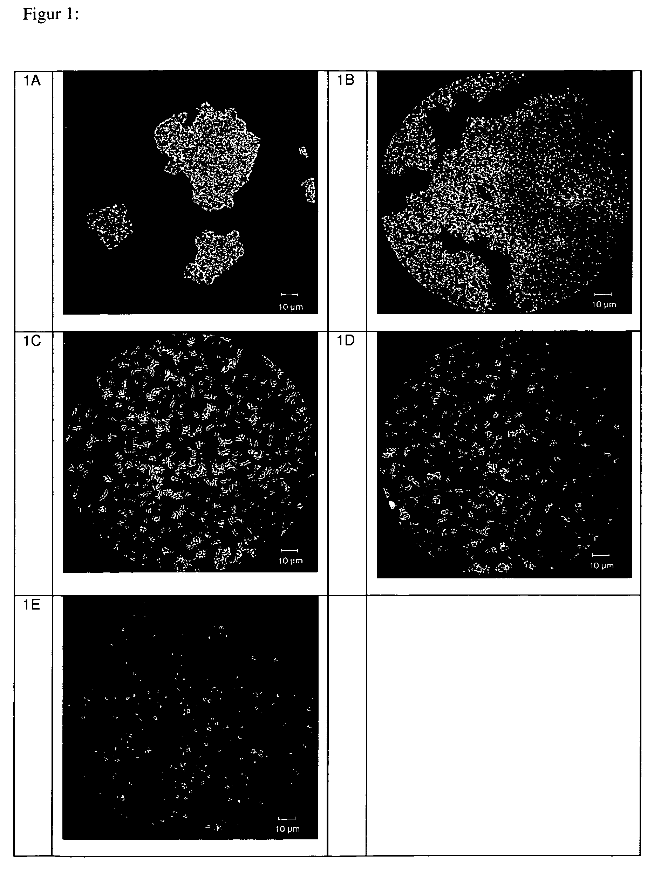

Figur 1A zeigt das mikroskopische Bild von Co-Aggregaten aus Helicobacter pylori und nicht-sprühgetrockneten Lactobacillus DSM 17648 Zellen. Figur 1B zeigt das mikroskopische Bild von Co-Aggregaten aus Helicobacter pylori und sprühgetrockneten Lactobacillus DSM 17648 Zellen. Diese Co-Aggregate sind deutlich größer als die Co-Aggregate mit nicht-sprühgetrockneten Lactobacillus Zellen. Kontrollexperimente auf Selbstaggregation wurden durch separate Untersuchung von Kulturen mit jeweils Lactobacillus und Helicobacter pylori allein durchgeführt. In den Figuren 1C, 1D und 1E ist weder Co-Aggregation noch Autoaggregation zu sehen. Figur 1C zeigt das mikroskopische Bild von frisch kultivierten Zellen von Lactobacillus DSM 17648 unter künstlichen Magenbedingungen. Figur 1D zeigt das mikroskopische Bild von sprühgetrockneten Zellen von Lactobacillus DSM 17648 unter künstlichen Magenbedingungen. Figur 1E zeigt das mikroskopische Bild von frisch kultivierten Helicobacter pylori Zellen unter künstlichen Magenbedingungen.

Beispiel 4: Testen der Stabilität der Co-Aggregate zwischen sprühgetrockneten Lactobacillus Zellen und Helicobacter pylori Zellen

Zum Testen der Stabilität der Co-Aggregate unter in vivo Bedingungen wurden die Experimente wie in Beispiel 3 durchgeführt. Nach 5 Minuten Schütteln gebildete Co-Aggregate zwischen Lactobacillus Zellen und Helicobacter pylori-Zellen wurden starken Scherkräften durch Pipettieren der Suspension für 1 Minute oder 2 minütiges Schütteln bei hoher Geschwindigkeit ausgesetzt. Danach wurde die Co-Aggregat-Größe mikroskopisch und makroskopisch untersucht. Die Größe der Co-Aggregate aus sprühgetrockneten Lactobacillus Zellen und Helicobacter pylori nahm nicht ab, wohingegen die Größe der Co-Aggregate aus nicht-sprühgetrockneten Lactobacillus Zellen und Helicobacter pylori abnahm.

Beispiel 5: Herstellung einer pharmazeutischen Zusammensetzung mit erfindungsgemäßen Lactobacillus Stämmen

Zellen eines Lactobacillus Stammes oder mehrerer Lactobacillus Stämme der Erfindung werden gemäß Beispiel 1 und 2 gezogen und sprühgetrocknet. Das sprühgetrocknete Pulver wird dann auf eine Partikelgröße von maximal ca. 1 mm Durchmesser gemahlen. Das erhaltene Granulat wird in den folgenden Mengenverhältnissen (Gew.-%) mit Träger- bzw. Hilfsstoffen gemischt: 20% Granulat, 2% Siliciumdioxid (Syloid AL-IFP, GRACE Davidson), 1% Magnesiumstearat (MF-2-V, Ackros), 77% mikrokristalline Zellulose (Avicel PH 112, FMC).

Das Mischen erfolgt in einem Quintech Micromixer bei Position 70 Level II. Alle Komponenten werden zugleich zugegeben. Die Mischung erfolgt für ca. 120 s. Anschließend wird die erhaltene Mischung in einer handelsüblichen Tablettenpresse unter üblichen Bedingungen, jedoch möglich niedriger Druckkraft (< 10 kN) zu Tabletten mit einem Gewicht von ca. 500 mg gepresst. Jede Tablette enthält ca. $10^8$ bis $10^{10}$ sprühgetrockneten Lactobacillus Zellen.

Beispiel 6: Vergleich der Co-Aggregation von Helicobacter pylori durch nicht sprühgetrocknete und sprühgetrocknete Lactobacillus Stämme bzw. Lactobacillus Zellen bei Einsatz von verschiedenen Zellzahlen des Lactobacillus.

Die Kultivierung der Lactobacillus Zellen erfolgte in geschlossenen 15 ml Röhrchen in MRS Medium bei 37 °C für 24 h. Für die Untersuchung von sprühgetrockneten Lactobacillus Zellen wurde das sprühgetrocknete Pulver in PBS mit einer Konzentration von 10 mg / ml resuspendiert. Helicobacter pylori wurde für ca. 2 Tage in Erlenmeyerkolben unter mikroaerophilen Bedingungen in Brucella Bouillon (28 g / l, pH 7,0; BD, USA) mit 10 % fötalem Kälberserum (Biochrom) bei 37 °C kultiviert. Nach der Kultivierung wurde die Zellmorphologie von Helicobacter pylori mikroskopisch untersucht. Es wurden Assays mit Zellen sigmoidaler Morphologie oder mit Zellen kokkoider Morphologie durchgeführt. Auch Kulturen mit gemischter Morphologie wurden untersucht.

Die jeweiligen Zellen wurden durch Zentrifugation bei 3.200 g für 10 min. geerntet und der Überstand wurde verworfen. Die Lactobacillus Zellen wurden einmal in 5 ml Puffer gewaschen und in 5 ml PBS Puffer resuspendiert (PBS-Puffer enthaltend 1,5 g/l $Na_2HPO_4*2H_2O$, 0,2 g/l $KH_2PO_4$ und 8,8 g/l NaCl). Die Helicobacter pylori Zellen wurden einmal in 5 ml PBS Puffer gewaschen und in 5 ml künstlichem Magensaft resuspendiert (enthaltend 5 g/l NaCl und 3 g/l Pepsin (Sigma)). Der OD600 Wert wurde für die jeweiligen Zellen gemessen und auf einen Wert von 2 für Helicobacter pylori bzw. 4 für Lactobacillus durch Zugabe von künstlichem Magensaft und PBS Puffer eingestellt.

Von den Lactobacillus-Suspensionen wurde mittels Thomakammer eine Zellzahlbestimmung durchgeführt und Verdünnungen hergestellt, um Suspensionen mit verschiedenen definierten Zellzahlen zu erhalten. 2,5 ml jeder so erhaltenen Zellsuspension (Helicobacter pylori / Lactobacillus) wurden gemischt und die Mischung wurde für 5 min geschüttelt. Nach einer Standzeit von 2 Minuten war das Ergebnis sowohl optisch sichtbar durch deutliche Flockulation in den Proben, die Helicobacter pylori und Lactobacillus enthielten, als auch mikroskopisch (Bilder nicht gezeigt). Zur Quantifizierung der Co-Aggregation wurde von der Co-Aggregat-freien Phase (Überstand; Co-Aggregate sedimentieren) 1 ml abgenommen und die OD600 bestimmt. Über die Formel

$$\text{Co-Aggregationsstärke \%} = \frac{(OD_{\text{H. pylori}} + OD_{\text{Lactobacillus}}) - OD_{\text{H.p.+Lactobacillus}}}{OD_{\text{H. pylori}} + OD_{\text{Lactobacillus}}} \times 100$$

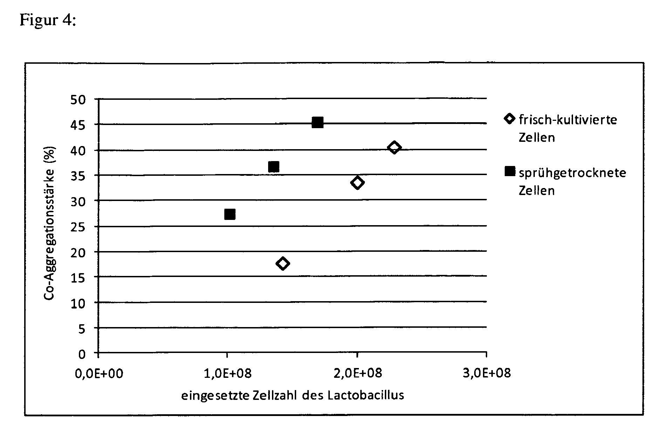

wurde die Co-Aggregationsstärke (in %) bestimmt. Figur 3 und 4 zeigt die Co-Aggregationsstärke von frisch-kultivierten und sprühgetrockneten Lactobacillus DSM 17648 Zellen gegenüber Helicobacter pylori. 0 % repräsentiert die OD600 des Helicobacter pylori (im Überstand) ohne Zugabe von Lactobacillus Zellen (keine Co-Aggregation bzw. keine durch Co-Aggregation bedingte Sedimentation). 100 % Aggregationsstärke repräsentiert eine maximale Co-Aggregationsstärke, also eine OD600 von 0 bzw. die OD600 des verwendeten Mediums ohne Zellen (sämtliche Helicobacter pylori Zellen sedimentieren bedingt durch Co-Aggregation und keine Helicobacter pylori Zellen sind im Überstand über OD Messung zu erfassen).

Bei gleicher Zellzahl weisen die sprühgetrockneten Lactobacillus-Zellen eine mehr als doppelt so starke Co-Aggregation von Helicobacter pylori auf als die frisch-kultivierten Zellen (Figur 3 und 4).

Beschreibung der Figuren:

Figur 1A zeigt Co-Aggregate aus frisch kultivierten nicht-sprühgetrockneten Lactobacillus DSM 17648 Zellen und Helicobacter pylori (Co-Aggregate zeigen eine Größe von 40 $\mu$m und mehr) unter simulierten Magenbedingungen.

Figur 1B zeigt ein sehr großes Co-Aggregat aus sprühgetrockneten Lactobacillus DSM 17648 Zellen und Helicobacter pylori (Co-Aggregate zeigen eine Größe von 150 $\mu$m und mehr) unter simulierten Magenbedingungen.

Figuren 1C bis 1E dienen als Kontrollen im Experiment.

Figur 1C zeigt frisch kultivierte Lactobacillus DSM 17648 Zellen unter simulierten Magenbedingungen. Eine Autoaggregation ist nicht erkennbar.

Figur 1D zeigt sprühgetrocknete Lactobacillus DSM 17648 Zellen unter simulierten Magenbedingungen. Eine Autoaggregation ist nicht erkennbar.

Figur 1E zeigt frisch kultivierte Helicobacter pylori Zellen unter simulierten Magenbedingungen. Eine Autoaggregation ist nicht erkennbar.

(Figur 1A-E sind 1.000-fach vergrößert)

Figuren 2A und 2B zeigen die Morphologie der Lactobacillus Zellen.

Figur 2A zeigt nicht-sprühgetrocknete Lactobacillus Zellen. Die Zellen liegen in Ketten von 2 bis 10 Zellen vor.

Figur 2B zeigt sprühgetrocknete Lactobacillus Zellen. Die Zellen liegen als Mono- und Dimere vor (1 bis 2 Zellen) und sind gegenüber nicht-sprühgetrockneten Zellen stark verkleinert.

Figur 3: Vergleich der Co-Aggregationsstärke von frischer Kultur und sprühgetrockneten Zellen von Lactobacillus DSM 17648 gegenüber Helicobacter pylori. Es wurden jeweils 1,4 x 10$^8$ Lactobacillus-Zellen in den Tests eingesetzt. Bei gleicher Zellzahl weisen die sprühgetrockneten Lactobacillus-Zellen eine mehr als doppelt so starke Co-Aggregationsaktivität von Helicobacter pylori auf als die frisch-kultivierten Zellen. 0 % repräsentiert die OD600 des Helicobacter pylori (im Überstand) ohne Zugabe von Lactobacillus Zellen (keine Co-Aggregation bzw. keine durch Co-Aggregation bedingte Sedimentation). 100 % Aggregationsstärke repräsentiert eine maximale Co-Aggregationsstärke, also eine OD600 von 0 bzw. die OD600 des verwendeten Mediums ohne Zellen (sämtliche Helicobacter pylori Zellen sedimentieren bedingt durch Co-Aggregation und keine Helicobacter pylori Zellen sind im Überstand über OD Messung zu erfassen).

Figur 4: Vergleich der Co-Aggregationsstärke von frischer Kultur und sprühgetrockneten Zellen von Lactobacillus DSM 17648 gegenüber Helicobacter pylori. Es wurden jeweils verschiedene Mengen an Lactobacillus-Zellen in den Tests eingesetzt. Die sprühgetrockneten Lactobacillus-Zellen weisen eine doppelt so starke Co-Aggregation von Helicobacter pylori auf als die frisch-kultivierten Zellen. 0 % repräsentiert die OD600 des Helicobacter pylori (im Überstand) ohne Zugabe von Lactobacillus Zellen (keine Co-Aggregation bzw. keine durch Co-Aggregation bedingte Sedimentation). 100 % Aggregationsstärke repräsentiert eine maximale Co-Aggregationsstärke, also eine OD600 von 0 bzw. die OD600 des verwendeten Mediums ohne Zellen (sämtliche Helicobacter pylori Zellen sedimentieren bedingt durch Co-Aggregation und keine Helicobacter pylori Zellen sind im Überstand über OD Messung zu erfassen).

**Patentansprüche**

1.  Zusammensetzung enthaltend sprühgetrocknete Lactobacillus Zellen zur Verwendung in der Behandlung und Prophylaxe von Helicobacter pylori Infektionen an Mensch oder Tier.

**2.** Zusammensetzung zur Verwendung nach Anspruch 1 enthaltend sprühgetrocknete Lactobacillus Zellen, **dadurch gekennzeichnet, dass** diese im Wesentlichen in einer mono und/oder dimeren Form vorliegen.

**3.** Zusammensetzung zur Verwendung nach Anspruch 1 oder 2, wobei a.) Lactobacillus Zellen sprühgetrocknet werden und b.) erhaltene sprühgetrocknete Lactobacillus Zellen in einen physiologisch verträglichen Träger eingebracht werden.

**4.** Zusammensetzung zur Verwendung nach einem der vorstehenden Ansprüche, wobei nach Applikation im Magenmedium in-situ Co-Aggregate mit Helicobacter pylori gebildet werden.

**5.** Zusammensetzung zur Verwendung nach einem der vorstehenden Ansprüche, wobei die Co-Aggregate nicht kleiner als 50 $\mu$m, insbesondere größer als 500 $\mu$m sind.

**6.** Zusammensetzung zur Verwendung nach einem der vorstehenden Ansprüche, wobei die Lactobacillus Zellen ausgewählt sind aus der Gruppe *Lactobacillus lactis, Lactobacillus helveticus, Lactobacillus jensenii, Lactobacillus acidophilus, Lactobacillus bulgaricus, Lactobacillus amylovorus, Lactobacillus delbrueckii, Lactobacillus casei, Lactobacillus crispatus, Lactobacillus gasseri, Lactobacillus johnsonii, Lactobacillus paracasei, Lactobacillus pentosus, Lactobacillus rhamnosus, Lactobacillus curvatus* und *Lactobacillus plantarum, Lactobacillus brevis, Lactobacillus buchneri, Lactobacillus fructivorans, Lactobacillus hilgardii, Lactobacillus fermentum, Lactobacillus reuteri, Lactobacillus viridescens,* DSM 17646, DSM 17647, DSM 17648, DSM 17649, DSM 17650, DSM 17651, DSM 17652, DSM 17653, vorzugsweise *Lactobacillus fermentum, Lactobacillus brevis, Lactobacillus reuteri, Lactobacillus buchneri* und *Lactobacillus pentosus.*

**7.** Zusammensetzung zur Verwendung nach einem der vorstehenden Ansprüche, **dadurch gekennzeichnet, dass** eine pharmazeutische oder diätische Zusammensetzung vorliegt, insbesondere in Form eines Nahrungs- bzw. Lebensmittels und/oder eines Futtermittels für Haus- und/oder Nutztiere und / oder Nahrungsergänzungsmittel, ggfs. enthaltend Hilfs- und Zusatzstoffe.

**8.** Zusammensetzung zur Verwendung nach einem der vorstehenden Ansprüche zur Eradikationstherapie von Helicobacter pylori, ggfs. in Kombination mit weiteren geeigneten Wirkstoffen, wie Antibiotika.

**9.** Arzneimittel enthaltend sprühgetrocknete Lactobacillus Zellen oder eine Zusammensetzung nach Anspruch 1 zur Verwendung in der Behandlung und Prophylaxe von Helicobacter pylori Infektionen an Mensch oder Tier.

**10.** Nahrungsergänzungsmittel enthaltend sprühgetrocknete Lactobacillus Zellen oder eine Zusammensetzung nach Anspruch 1 zur Verwendung bei Helicobacter pylori Infektionen an Mensch oder Tier.

**11.** Arzneimittel oder Nahrungsergänzungsmittel oder diätätische Zusammensetzung enthaltend sprühgetrocknete Lactobacillus Zellen zur Verwendung bei Infektionen mit Helicobacter pylori bedingten Erkrankungen, insbesondere Gastrointestinalerkrankungen, wie Gastritis, Magengeschwüre, Ulcus und Magenkrebs, Bauchbeschwerden, insbesondere Oberbauchbeschwerden, Magendruck, Magenkrämpfe, Magenschmerzen, Schmerzen oder Druck im Oberbauch, Brennen im Oberbauch, chronisch-rezidivierende Abdominalbeschwerden, ständiges Völlegefühl, Appetitlosigkeit, Nüchternschmerz, Blähungen, Sodbrennen, Durchfall, Stuhlunregelmäßigkeiten, Unwohlsein, Brechreiz, Übelkeit und Erbrechen, Speiseunverträglichkeiten, Malabsorption, Reizmagen (funktionelle Dyspepsie), Magenschleimhautentzündung, Schleimhautschädigung, gastroduodenale Ulkuskrankheit, insbesondere deren Prophylaxe und Behandlung, und zur Verwendung in der Eradikationstherapie von Helicobacter pylori, ggfs. in Kombination mit weiteren geeigneten Wirkstoffen, wie Antibiotika.

**Claims**

**1.** A composition, comprising spray-dried Lactobacillus cells for use in the treatment and prophylaxis of Helicobacter pylori infections in humans or animals.

**2.** The composition for use according to claim 1, comprising spray-dried Lactobacillus cells, **characterized in that** these are essentially present in monomer and/or dimer form.

**3.** The composition for use according to claim 1 or 2, wherein a.) Lactobacillus cells are spray-dried, and b.) spray-dried Lactobacillus cells that are obtained are introduced into a physiologically compatible carrier.

4. A composition for use according to any one of the preceding claims, wherein co-aggregates are formed with Helicobacter pylori in situ after application in the stomach medium.

5. A composition for use according to any one of the preceding claims, wherein the co-aggregates are not smaller than 50 µm, and more particularly are larger than 500 µm.

6. A composition for use according to any one of the preceding claims, wherein the Lactobacillus cells are selected from the group consisting of *Lactobacillus lactis, Lactobacillus helveticus, Lactobacillus jensenii, Lactobacillus acidophilus, Lactobacillus bulgaricus, Lactobacillus amylovorus, Lactobacillus delbrueckii, Lactobacillus casei, Lactobacillus crispatus, Lactobacillus gasseri, Lactobacillus johnsonii, Lactobacillus paracasei, Lactobacillus pentosus, Lactobacillus rhamnosus, Lactobacillus curvatus* and *Lactobacillus plantarum, Lactobacillus brevis, Lactobacillus buchneri, Lactobacillus fructivorans, Lactobacillus hilgardii, Lactobacillus fermentum, Lactobacillus reuteri, Lactobacillus viridescens,* DSM 17646, DSM 17647, DSM 17648, DSM 17649, DSM 17650, DSM 17651, DSM 17652, DSM 17653, preferably *Lactobacillus fermentum, Lactobacillus brevis, Lactobacillus reuteri, Lactobacillus buchneri* and *Lactobacillus pentosus.*

7. A composition for use according to any one of the preceding claims, **characterized in that** a pharmaceutical or dietary composition is present, in particular in the form of a food or foodstuff and/or a feedstuff for pets and/or farm animals and/or dietary supplement, optionally comprising adjuvants and additives.

8. A composition for use according to any one of the preceding claims for eradication therapy of Helicobacter pylori, optionally in combination with further suitable active ingredients such as antibiotics.

9. A pharmaceutical product, comprising spray-dried Lactobacillus cells or a composition according to claim 1 for use in the treatment and prophylaxis of Helicobacter pylori infections in humans or animals.

10. A dietary supplement, comprising spray-dried Lactobacillus cells or a composition according to claim 1 for use with Helicobacter pylori infections in humans or animals.

11. A pharmaceutical product or dietary supplement or dietetic composition for use with conditions caused by infections with Helicobacter pylori, in particular gastrointestinal conditions such as gastritis, stomach ulcers and stomach cancer, abdominal discomfort, in particular discomfort of the upper abdomen, gastric heaviness, gastric spasms, stomach pain, pain or pressure in the upper abdomen, burning sensation in the upper abdomen, chronic-recurrent abdominal disorders, permanent feeling of fullness, lack of appetite, fasting pain, bloating, heartburn, diarrhea, irregular bowel movements, indisposition, nausea, sickness and vomiting, food intolerance, malabsorption, upset stomach (functional dyspepsia), gastritis, damage to the mucous membrane, or gastroduodenal ulcer in particular for the prophylaxis and the treatment thereof, and for use in the eradication therapy of Helicobacter pylori, optionally in combination with further suitable active ingredients such as antibiotics.

**Revendications**

1. Composition, comprenant des cellules de bacilles lactiques séchées par vaporisation pour une utilisation dans le traitement et la prévention d'infections par l'helicobacter pylori chez des humains ou des animaux.

2. Composition pour une utilisation selon la revendication 1, comprenant des cellules de bacilles lactiques séchées par vaporisation, **caractérisée en ce que** celles-ci sont essentiellement présent sous une forme monomère et/ou dimère.

3. Composition pour une utilisation selon la revendication 1, ou 2, dans laquelle a.) les cellules de bacilles lactiques sont séchées par vaporisation, et b.) les cellules de bacilles lactiques séchées par vaporisation qui sont obtenues sont introduites dans un vecteur physiologiquement compatible.

4. Composition pour une utilisation selon l'une quelconque des revendications précédentes, dans laquelle des co-agrégats sont formés in situ avec l'helicobacter pylori après l'application dans le milieu stomacal.

**5.** Composition pour une utilisation selon l'une quelconque des revendications précédentes, dans laquelle les co-agrégats ne sont pas plus petits que 50 μm, et sont plus particulièrement plus grands que 500 μm.

**6.** Composition pour une utilisation selon l'une quelconque des revendications précédentes, dans laquelle les cellules de bacilles lactiques sont choisies dans le groupe constitué de *Lactobacillus lactis, Lactobacillus helveticus, Lactobacillus jensenii, Lactobacillus acidophilus, Lactobacillus bulgaricus, Lactobacillus amylovorus, Lactobacillus delbrueckii, Lactobacillus casei, Lactobacillus crispatus, Lactobacillus gasseri, Lactobacillus johnsonii, Lactobacillus paracasei, Lactobacillus pentosus, Lactobacillus rhamnosus, Lactobacillus curvatus* et de *Lactobacillus plantarum, Lactobacillus brevis, Lactobacillus buchneri, Lactobacillus fructivorans, Lactobacillus hilgardii, Lactobacillus fermentum, Lactobacillus reuteri, Lactobacillus viridescens,* DSM 17646, DSM 17647, DSM 17648, DSM 17649, DSM 17650, DSM 17651, DSM 17652, DSM 17653, de préférence de *Lactobacillus fermentum, Lactobacillus brevis, Lactobacillus reuteri, Lactobacillus buchneri* et de *Lactobacillus pentosus.*

**7.** Composition pour une utilisation selon l'une quelconque des revendications précédentes, **caractérisée en ce qu'**une composition pharmaceutique ou diététique est présente, en particulier sous la forme d'un aliment ou d'une denrée alimentaire et/ou d'un aliment pour des animaux de compagnie et/ou des animaux de ferme et/ou d'un complément alimentaire, comprenant éventuellement des adjuvants et des additifs.

**8.** Composition pour une utilisation selon l'une quelconque des revendications précédentes, pour une thérapie d'éradication de l'helicobacter pylori, éventuellement en combinaison avec d'autres ingrédients actifs appropriés tels que des antibiotiques.

**9.** Produit pharmaceutique, comprenant des cellules de bacilles lactiques séchées par vaporisation ou composition selon la revendication 1 pour une utilisation dans le traitement et la prévention d'infections par l'helicobacter pylori chez les humains ou les animaux.

**10.** Complément diététique, comprenant des cellules de bacilles lactiques séchées par vaporisation ou composition selon la revendication 1 pour une utilisation avec des infections par l'helicobacter pylori chez les humains ou les animaux.

**11.** Produit pharmaceutique ou complément diététique ou composition diététique pour une utilisation dans des troubles causés par des infections avec l'helicobacter pylori, en particulier des troubles gastro-intestinaux tels que la gastrite, les ulcères de l'estomac et le cancer de l'estomac, l'inconfort abdominal, en particulier l'inconfort de l'abdomen supérieur, la lourdeur gastrique, les spasmes gastriques, la douleur stomacale, la douleur ou la pression dans l'abdomen supérieur, la sensation de brûlure dans l'abdomen supérieur, les troubles abdominaux chroniques récurrents, une sensation permanente d'être totalement rassasié, un manque d'appétit, une douleur de jeûne, les ballonnements, les brûlures d'estomac, la diarrhée, les mouvements de l'intestin irréguliers, l'indisposition, la nausée, la fatigue et les vomissements, l'intolérance alimentaire, la malabsorption, l'estomac retourné (dyspepsie fonctionnelle), la gastrite, l'endommagement de la membrane muqueuse, ou l'ulcère gastroduodénal, en particulier pour la prévention et le traitement de ceux-ci, et pour une utilisation dans la thérapie d'éradication de l'helicobacter pylori, éventuellement en combinaison avec d'autres ingrédients actifs appropriés tels que des antibiotiques.

Figur 1:

Figur 2:

Figur 3:

Figur 4:

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5716615 A
- WO 2004087891 A
- EP 1112692 A1
- WO 2008060198 A1
- WO 2007073709 A

**In der Beschreibung aufgeführte Nicht-Patentliteratur**

- **WANG et al.** *Am. J. Clin. Nutr.,* 2004, vol. 80, 737-41
- **FELLEY et al.** *Best Practice & Research Clinical Gastroenterology,* 2003, vol. 17 (5), 785-791
- **CAZZATO et al.** *Scandinavian Journal of Nutrition,* 2004, vol. 48 (1), 26-31
- **SGOURAS et al.** *Applied and Environmental Microbiology,* 2004, vol. 70 (1), 518-526
- **GARDINER et al.** Comparative Survival Rates of Human-Derived Probiotic Lactobacillus paracasei and L. salivarius Strains during Heat Treatment and Spray Drying. *Applied and Enviromental Microbiology,* 2000, vol. 66 (6), 2605-2612
- **TEIXEIRA et al.** Survival of Lactobacillus delbrueckii ssp. Bulgaricus Following Spray-Drying. *J. Dairy Sci,* 1995, vol. 78, 1025-1031
- Spray Drying, Freeze Drying, or Freezing of three different lactic acid bacteria species. **TOS T.** Journal of Food Sci. Wiley-Blackwell Publ., Inc,
- **SCHLEIFER et al.** *System. Appl. Microb.,* 1995, vol. 18, 461-467
- **LUDWIQ et al.** *System. Appl. Microb.,* 1992, vol. 15, 487-501
- **J. SAMBROOK ; E.F. FRITSCH ; T. MANIATIS.** Cold Molecular cloning: a laboratory manual. Spring Harbor Laboratory Press, 2001